# FASCICULE DE BREVET EUROPEEN

(11) **EP 3 562 950 B1**
(45) Date de publication et mention de la délivrance du brevet: **02.10.2024**
(21) Numéro de dépôt: 17832529.6
(22) Date de dépôt: 21.12.2017
(51) Int. Cl.: C12P 17/16, C07D 277/16, C07D 279/06

(54) **PROCÉDÉ DE SYNTHÈSE DE DITHIOCARBAMATES CYCLIQUES FONCTIONNALISÉS**
VERFAHREN ZUR SYNTHESE VON FUNKTIONALISIERTEN CYCLISCHEN DITHIOCARBAMATEN
PROCESS FOR THE SYNTHESIS OF FUNCTIONALIZED CYCLIC DITHIOCARBAMATES

(30) Priorité: 29.12.2016 FR 1663491
(43) Date de publication de la demande: 06.11.2019
(73) Titulaire: Arkema France, 92700 Colombes (FR)
(72) Inventeur: FREMY, Georges, 64390 Sauveterre de Bearn (FR); MASSELIN, Arnaud, 35400 Saint Malo (FR)
(74) Mandataire: Bandpay & Greuter
(86) Numéro de dépôt international: PCT/FR2017/053781
(87) Numéro de publication internationale: WO 2018/122510

(56) Documents cités:
- EP-A2- 0 323 068
- WO-A1-2008/013432
- US-A- 3 960 881
- HASE ET AL: "The growth inhibitors from a few plants", REVISTA LATINOAMERICANA DE QUIMICA, MONTERREY, MX, vol. 16, no. 1, 1 January 1985 (1985-01-01), pages 1 - 5, XP009525677, ISSN: 0370-5943
- JOHNSON D J ET AL: "The Measurement of 2-Thiothiazolidine-4-carboxylic Acid as an Index of the in Vivo Release of CS 2 by Dithiocarbamates", vol. 9, 15 June 1996 (1996-06-15), pages 910 - 916, XP055776348, Retrieved from the Internet <URL:https://pubs.acs.org>
- ALLISON JOBSON ET AL: "(4R)-(-)-2-Thioxothiazolidine-4-carboxylic Acid (Raphanusamic Acid)", ACTA CRYSTALLOGRAPHICA SECTION C, vol. C54, 1998, pages 1634 - 1637, XP002774215
- ENTESAR A.HASSAN ET AL: "Dithiocarbamates as precursors in organic chemistry; Synthesis and uses", PHOSPHORUS,SULFUR,AND SILICON, vol. 189, 2014, pages 300 - 323, XP002774216
- DATABASE CAPLUS [online] CHEMICAL ABSTRACTS SERVICE, COLUMBUS, OHIO, US; 1995, HUANG, HUA-MIN ET AL: "Synthesis of (R)-2-thioxothiazolidine-4-carboxylic acid and its esters", XP002774217, accession no. 1995:436361
- HUANG, HUA-MIN ET AL: "Synthesis of (R)-2thioxothiazolidine-4-carboxylicacid and its esters", YOUJI HUAXUE, vol. 15, no. 1, 1995, pages 67 - 71
- WAEL M. RABEH ET AL: "Structure and Mechanism of O -Acetylserine Sulfhydrylase", JOURNAL OF BIOLOGICAL CHEMISTRY, vol. 279, no. 26, 25 June 2004 (2004-06-25), US, pages 26803 - 26806, XP055407135, ISSN: 0021-9258, DOI: 10.1074/jbc.R400001200

## Description

### Domaine de l'invention

L'invention concerne le domaine des dithiocarbamates cycliques et plus spécifiquement un procédé de synthèse de dithiocarbamates cycliques, et plus particulièrement de dithiocarbamates cycliques fonctionnalisés.

### Arrière-plan technique

Les dithiocarbamates sont des composés organiques pouvant être utilisés en tant que précurseurs de radicaux, intermédiaires de synthèse organique, agents de vulcanisation, chélateurs ou inhibiteurs d'enzyme. Leurs domaines d'application sont divers et ils peuvent par exemple entrer dans la composition de fongicides, de désherbants, de pesticides ou d'insecticides dans le domaine agricole, être utilisés dans l'industrie du caoutchouc ou encore dans l'industrie pharmaceutique dans le traitement de maladies telles que le cancer ou le VIH.

Par exemple, l'article de Allison Dobson et al., « (4R)-(-)-2-Thioxothiazolidine-4-carboxylic Acid (Raphanusamic Acid) », ACTA CRYSTALLOGRAPHICA SECTION C, vol. C54, 1998, pp. 1634-1637, décrit l'acide (4R)-(-)2-thioxothiazolidine-4-carboxylique et sa structure absolue.

De par la multitude d'applications des dithiocarbamates, il existe diverses techniques de synthèses de ces composés.

Ainsi, l'article de Entesar A. Hassan, « Dithiocarbamates as precursors in organic chemistry, synthesis and uses », Phosphorus, Sulfur and Silicon, vol. 189, (2014), pp. 300-323, décrit différents procédés de synthèse des dithiocarbamates. Il est par exemple divulgué la synthèse de N,N-dialkyldithiocarbamate par réaction d'un sel de dithiocarbamate avec un halogénure d'alkyle ou avec un phosphate de dialkyle, ou par addition d'oléfines électron-déficientes. Cet article décrit également l'obtention de dithiocarbamates par acylation d'amines par du chlorodithioformate. Il est aussi divulgué la préparation de dithiocarbamates cycliques par réaction de disulfures avec (i) du 2-amino-éthanol, du sulfate de 2-aminoéthyle et un halogénure de 2-aminoéthyle, (ii) des amines primaires et du 1,2-dibromoéthane en présence d'une base, (iii) des aziridines et (iv) des 2-iminothiazolidines. Les dithiocarbamates cycliques peuvent également être préparés par cyclisation de β-hydroxyalkyldithiocarbamates par traitement avec du chlorure de mésyle dans de la pyridine, ou encore par cyclisation du 2-alkylaminoéthanethiol avec du thiophosgène en présence d'une base.

Par ailleurs, la demande CN 103804258 décrit la synthèse de dithiocarbamates à partir de carbamide et de disulfure de carbone et la demande CN 103804257 divulgue la préparation de diéthyldithiocarbamates également à partir de carbamide et de disulfure de carbone.

Quant à la demande US 2046876, cette dernière décrit la synthèse de N-diaryl-dithiocarbamates par ajout de disulfure de carbone sur des dérivés de diarylamines.

On comprendra aisément que, de par la multitude des applications possibles des dithiocarbamates, il existe un besoin toujours croissant pour de nouveaux dithiocarbamates, en particulier de nouveaux dithiocarbamates fonctionnalisés, tout particulièrement pour des applications en polymérisation (notamment pour un greffage éventuel sur un support organique), pour des applications en dévulcanisation et autres, mais aussi, et plus spécifiquement, de nouveaux dithiocarbamates optiquement actifs pour les très nombreuses applications en pharmacie, biologie, et autres. L'article de Huang et al., « Synthesis of (R)-2-thioxothiazolidine-4-carboxylic acid and its esters », DATABASE CAPLUS CHEMICAL ABSTRACTS SERVICE, COLUMBUS, OHIO, US; 1995, 1995:436361, décrit la synthèse de l'acide (R)-2-thioxothiazolidine-4-carboxylique à partir du chlorhydrate de L-cystéine monohydraté et du disulfure de carbone dans une solution aqueuse d'hydroxyde de sodium et de nitrate de plomb. La demande US 3960881 décrit des composés de thiazolidine et de thiazine-2-thione, dont la 4-carboxythiazolidine-2-thione et la 3,4-tétraméthylène-4H,5,6-dihydro-1,3-thiazine-2-thione, ainsi que leurs méthodes de préparation. L'article de Tsunao Hase, « The growth inhibitors from a few plants », Rev. Latinoamer. Quim., 16-1, 1-5 (1985): XP9525677 décrit la synthèse des acides raphanusamique et homoraphanusamique. L'article de D. J. Johnson et al., « The Measurement of 2-Thiothiazolidine-4-carboxylic Acid as an Index of the in Vivo Release of CS2 by Dithiocarbamates », Chem. Res. Toxicol., 1996, 9, 910-916: XP55776348, décrit l'obtention de l'acide 2-thiothiazolidine-4-carboxylique (TTCA) à partir d'un dithiocarbamate.

On comprendra aussi par la description des méthodes de synthèse qu'il existe également un besoin pour des synthèses de dithiocarbamates avec des procédés que l'on peut qualifier de durables, c'est-à-dire réalisables avec des conditions douces de température et de pression, en solution aqueuse avec des pH proches de la neutralité, et avec des matières premières d'origine renouvelable, et plus généralement plus respectueuses de l'environnement.

Il a maintenant été découvert qu'il est possible de répondre aux objectifs définis ci-dessus, en totalité ou au moins en partie, en mettant en oeuvre le procédé selon l'invention et tel que décrit ci-après. D'autres objectifs encore apparaîtront dans la suite de la description de la présente invention qui suit.

### Résumé de l'invention

L'invention concerne un procédé de synthèse d'un dithiocarbamate cyclique fonctionnalisé tel que défini dans la revendication 1. Des modes de réalisation sont présentés dans les revendications 2 à 11. L'invention concerne également un acide L-homoraphanusamique tel que défini dans la revendication 12.

### Description détaillée

Ainsi, et selon un premier aspect, la présente invention concerne un procédé de synthèse d'un dithiocarbamate cyclique fonctionnalisé de formule (I) : 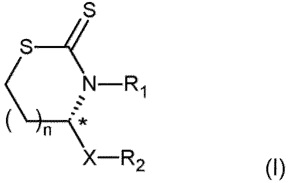 dans laquelle
- R₁ est un hydrogène ou une chaîne hydrocarbonée ramifiée ou non, saturée ou insaturée, linéaire ou cyclique, aromatique ou non, comportant de 1 à 20 atomes de carbone, et pouvant comporter un ou plusieurs hétéroatomes choisis parmi O, S, N, P et Si ;
- X représente -C(=O)- ou -CH₂- ou -CN ;
- R₂ est (i) soit nul (quand X représente -CN), (ii) soit un hydrogène, (iii) soit -OR₃, R₃ étant un hydrogène ou une chaîne hydrocarbonée ramifiée ou non, saturée ou insaturée, linéaire ou cyclique, aromatique ou non, comportant de 1 à 20 atomes de carbone, et pouvant comporter ou plusieurs hétéroatomes choisis parmi O, S, N, P et Si, (iv) soit -NR₄R₅ avec R₄ et R₅, différents ou non, étant un hydrogène ou une chaîne hydrocarbonée ramifiée ou non, saturée ou insaturée, linéaire ou cyclique, aromatique ou non, comportant de 1 à 20 atomes de carbone, et pouvant comporter un ou plusieurs hétéroatomes choisis parmi O, S, N, P et Si ;
- n est égal à 0, 1 ou 2, de préférence 1 ; et
- * représente un carbone asymétrique,

ledit procédé comprenant les étapes de :
   a/ fourniture d'au moins un composé de formule (II) :

      G-(CH₂)ₙ-C*H(NHR₁)-X-R₂ (II)

      dans laquelle
      - n, R₁, R₂, X, et * sont tels que définis précédemment,
      - G représente soit (i) R₆-C(=O)-O-CH₂-, soit (ii) (R₇O)(R₈O)-P(=O)-O-CH₂-, soit (iii) R₇O-SO₂-O-CH₂- ;
      - R₆ est un hydrogène ou une chaîne hydrocarbonée ramifiée ou non, saturée ou insaturée, linéaire ou cyclique, aromatique ou non, comportant de 1 à 20 atomes de carbone, et pouvant comporter un ou plusieurs hétéroatomes choisis parmi O, S, N, P et Si ;
      - R₇ et R₈, identiques ou différents, sont choisis indépendamment l'un de l'autre parmi un proton H, un alcalin, un alcalino-terreux ou un ammonium, de préférence un proton H ou un alcalin, et plus particulièrement un proton H ou Na,
   b/ fourniture d'au moins un trithiocarbonate inorganique,
   c/ réaction entre ledit au moins composé de formule (II) et ledit au moins trithiocarbonate inorganique en présence d'au moins une enzyme choisie parmi les sulfhydrylases, et de préférence une sulfhydrylase associée audit composé de formule (II),
   d/ obtention d'au moins un dithiocarbamate cyclique fonctionnalisé de formule (I),
   e/ séparation et isolation dudit au moins un dithiocarbamate cyclique fonctionnalisé de formule (I),
   f/ de façon optionnelle, fonctionnalisation supplémentaire du dithiocarbamate cyclique fonctionnalisé de formule (I) obtenu à l'étape d/ ou e/,
les étapes a/ et b/ étant, ou non, effectuées de manière simultanée.

Il a été observé que la configuration du carbone asymétrique est conservée tout au long de la réaction. Comme autre avantage, il est à noter que le dithiocarbamate cyclique fonctionnalisé de formule (I) obtenu selon le procédé selon l'invention est un dithiocarbamate énantiomériquement pur.

Par « dithiocarbamate cyclique fonctionnalisé », on entend tout type de dithiocarbamate cyclique de formule (I) dont l'atome d'azote porte un groupement fonctionnel (sauf lorsque R₁ représente l'atome d'hydrogène) et/ou l'atome de carbone en alpha de l'atome d'azote porte un groupement fonctionnel (sauf lorsque -X-représente -CH₂- et que R₂ l'atome d'hydrogène).

L'invention sera mieux comprise au regard de la description et des exemples qui suivent mais n'est en aucun cas limitée aux dits exemples.

Selon un mode de réalisation de l'invention, R₁ représente l'atome d'hydrogène.

Selon un autre mode réalisation de l'invention, -X- représente -C(=O)-.

Selon encore un autre mode de réalisation de l'invention, R₂ représente -OR₃ avec R₃ étant l'atome d'hydrogène.

Selon un autre mode de réalisation de l'invention, n est égal à 0.

Selon encore un autre mode de réalisation de l'invention, n est égal à 1.

Selon un mode de réalisation préféré de l'invention, au sein de la formule (I), R₁ représente l'atome d'hydrogène, -X- représente -C(=O)-, -R₂ représente -OR₃ avec R₃ étant un hydrogène, n est égal à 0, et le composé de formule (I) est l'acide L-raphanusamique.

Selon un autre mode de réalisation préféré de l'invention, au sein de la formule (I), R₁ représente l'atome d'hydrogène, -X- représente -C(=O)-, -R₂ représente -OR₃ avec R₃ étant l'atome d'hydrogène, n est égal à 1, et le composé de formule (I) est l'acide L-homoraphanusamique.

Selon un mode de réalisation préféré de l'invention, au sein de la formule (II), R₁ représente l'atome d'hydrogène, -X- représente -C(=O)-, -R₂ représente -OR₃ avec R₃ étant un hydrogène, n est égal à 0, et le composé de formule (II) est un dérivé de la L-sérine.

Le dérivé de la L-sérine utilisé dans le procédé selon l'invention peut, par exemple et de manière non limitative, être choisi parmi l'O-phospho-L-sérine, l'O-succinyl-L-sérine, l'O-acétyl-L-sérine, l'O-acétoacétyl-L-sérine, la O-propio-L-sérine, la O-coumaroyl-L-sérine, la O-malonyl-L-sérine, l'O-hydroxyméthylglutaryl-L-sérine, la O-pimélyl-L-sérine et la O-sulfato-L-sérine.

De manière préférée, le dérivé de L-sérine est choisi parmi l'O-phospho-_{L}-sérine, l'O-succinyl-L-sérine, l'O-acétyl-L-sérine et la O-sulfato-L-sérine.

De manière tout particulièrement préférée, le dérivé de L-sérine est l'O-acétyl-L-sérine.

Selon un autre mode de réalisation préféré de l'invention, au sein de la formule (II), R₁ représente l'atome d'hydrogène, X représente la fonction C=O, R₂ représente -OR₃ avec R₃ étant un hydrogène, n est égal à 1, et le composé de formule (II) est un dérivé de la L-homosérine.

Le dérivé de L-homosérine utilisé dans le procédé selon l'invention peut, par exemple et de manière non limitative, être choisi parmi l'O-phospho-L-homosérine, l'O-succinyl-L-homosérine, l'O-acétyl-L-homosérine, l'O-acétoacétyl-L-homosérine, l'O-propio-L-homosérine, l'O-coumaroyl-L-homosérine, l'O-malonyl-L-homosérine, l'O-hydroxyméthylglutaryl-L-homosérine, l'O-pimélyl-L-homosérine et l'O-sulfato-_{L}-homosérine.

De manière préférée, le dérivé de _{L}-homosérine est choisi parmi l'O-phospho-L-homosérine, l'O-succinyl-L-homosérine, l'O-acétyl-L-homosérine et l'O-sulfato-_{L}-homosérine.

De manière tout particulièrement préférée, le dérivé de _{L}-homosérine est l'O-acétyl-L-homosérine (OAHS).

Le dérivé de la L-sérine et le dérivé de la L-homosérine sont soit disponibles dans le commerce, soit obtenus par toute technique connue de l'homme du métier.

Ils peuvent par exemple être obtenus par fermentation d'une matière première renouvelable. La matière première renouvelable peut être choisie parmi le glucose, le saccharose, l'amidon, la mélasse, le glycérol, le bioéthanol, de préférence le glucose.

Le dérivé de la L-sérine peut également être produit à partir de l'acétylation de la L-sérine, la L-sérine, pouvant elle-même être obtenue par fermentation d'une matière première renouvelable. La matière première renouvelable peut être choisi parmi le glucose, le saccharose, l'amidon, la mélasse, le glycérol, le bioéthanol, de préférence le glucose.

Le dérivé de la _{L}-homosérine peut également être produit à partir de l'acétylation de la _{L}-homosérine, la _{L}-homosérine, pouvant elle-même être obtenue par fermentation d'une matière première renouvelable. La matière première renouvelable peut être choisie parmi le glucose, le saccharose, l'amidon, la mélasse, le glycérol, le bioéthanol, de préférence le glucose.

Le trithiocarbonate inorganique utilisé dans le procédé selon l'invention peut être choisi parmi un trithiocarbonate d'alcalin, un trithiocarbonate d'alcalino-terreux ou un trithiocarbonate d'ammonium.

De manière préférée, le trithiocarbonate inorganique est choisi parmi le trithiocarbonate de sodium, le trithiocarbonate de potassium, le trithiocarbonate de calcium et le trithiocarbonate d'ammonium.

De manière particulièrement préférée, le trithiocarbonate inorganique est le trithiocarbonate de sodium.

Au cours du procédé selon l'invention, la réaction entre ledit au moins composé de formule (II) et ledit au moins trithiocarbonate inorganique s'effectue en présence d'au moins une enzyme, ladite enzyme étant de préférence une sulfhydrylase associée au dit composé de formule (II).

Ainsi, lorsque le composé de formule (II) est un dérivé de la _{L}-sérine, l'enzyme utilisée peut être choisie parmi l'O-phospho-L-sérine sulfhydrylase, l'O-succinyl-L-sérine sulfhydrylase, l'O-acétyl-L-sérine sulfhydrylase, l'O-acétoacétyl-L-sérine sulfhydrylase, l'O-propio-L-sérine sulfhydrylase, l'O-coumaroyl-L-sérine sulfhydrylase, l'O-malonyl-L-sérine sulfhydrylase, l'O-hydroxyméthylglutaryl-L-sérine sulfhydrylase, l'O-pimélyl-L-sérine sulfhydrylase et l'O-sulfato-L-sérine sulfhydrylase.

De manière préférée, l'enzyme associée au dérivé de la L-sérine est choisie parmi l'O-phospho-L-sérine sulfhydrylase, l'O-succinyl-L-sérine sulfhydrylase, l'O-acétyl-L-sérine sulfhydrylase et l'O-sulfato-L-sérine sulfhydrylase.

De manière tout particulièrement préférée, l'enzyme associée au dérivé de la L-sérine est l'O-acétyl-L-sérine sulfhydrylase.

Par ailleurs, lorsque le composé de formule (II) est un dérivé de la _{L}-homosérine, l'enzyme qui utilisée peut être choisie parmi l'O-phospho-L-homosérine sulfhydrylase, l'O-succinyl-L-homosérine sulfhydrylase, l'O-acétyl-L-homosérine sulfhydrylase, l'O-acéto-acétyl-L-homosérine sulfhydrylase, l'O-propio-L-homosérine sulfhydrylase, l'O-coumaroyl-_{L}-homosérine sulfhydrylase, l'O-malonyl-L-homosérine sulfhydrylase, l'O-hydroxyméthyl-glutaryl-L-homosérine sulfhydrylase, l'O-pimélyl-L-homosérine sulfhydrylase et l'O-sulfato-_{L}-homosérine sulfhydrylase.

De manière préférée, l'enzyme associée au dérivé de la _{L}-homosérine est choisie parmi l'O-phospho-L-homosérine sulfhydrylase, l'O-succinyl-L-homosérine sulfhydrylase, l'O-acétyl-L-homosérine sulfhydrylase et l'O-sulfato-L-homosérine sulfhydrylase.

De manière tout particulièrement préférée, l'enzyme associé au dérivé de la _{L}-homosérine est l'O-acétyl-L-homosérine sulfhydrylase.

Ces dites enzymes présentent un fonctionnement optimal, comme cela est bien connu de l'homme du métier, lorsqu'elles sont utilisées en présence d'un cofacteur, tel que par exemple le pyridoxal-5'-phosphate (PLP).

L'enzyme et son cofacteur associé sont généralement dissous dans de l'eau avant d'être ajoutés au milieu réactionnel. La proportion d'enzyme par rapport à la masse du composé de formule (II), sera comprise entre 0,1% et 10% en poids, de préférence entre 1% et 5% en poids, et la quantité de cofacteur par rapport au composé de formule (II) sera comprise entre 0,1% et 10% en poids, de préférence entre 0,5% et 5% en poids.

En ce qui concerne les conditions de pH, de températures, de milieu de synthèse, on peut se référer à celles décrites dans les demandes WO2008013432 et WO2013029690.

Ainsi, le pH réactionnel est de préférence compris entre 5 et 8, de préférence entre 6 et 7,5, et plus particulièrement entre 6,2 et 7,2. Ledit pH est fonction du domaine de fonctionnement de l'enzyme et peut être régulé suivant l'optimum de l'enzyme, par ajout du trithiocarbonate basique ou en ajoutant de l'acide sulfurique dilué ou de l'ammoniaque dilué. De manière préférée, le pH est ajusté par régulation de l'ajout du trithiocarbonate basique.

Ainsi, la température au cours de la réaction est comprise entre 10°C et 45°C, de préférence entre 20°C et 40°C, et plus particulièrement entre 25°C et 35°C. Ladite température est choisie suivant le domaine de fonctionnement de l'enzyme.

La réaction se déroule en milieu aqueux ou en présence de solvants organiques si ces derniers sont compatibles avec les enzymes utilisées. De manière préférée, la réaction se déroule en milieu aqueux.

La réaction peut être menée en batch, en semi-continu ou en continu. Tout type de réacteurs, connu de l'homme de métier, peut convenir à ce type de réactions.

Selon un mode de réalisation de l'invention, la séparation et l'isolation du dithiocarbamate obtenu peuvent s'effectuer selon toute technique connue de l'homme du métier, en particulier par précipitation et filtration.

L'étape f/ optionnelle du procédé selon l'invention permet d'obtenir des fonctions supplémentaires et différentes de celles obtenues après l'étape d/ ou à l'étape e/.

En effet, le dithiocarbamate cyclique fonctionnalisé de formule (I) obtenu à l'issue de l'étape d/ ou à l'issue de l'étape e/ peut de nouveau être fonctionnalisé au cours de cette étape f/. Par exemple, si X-R₂ représente une fonction carboxylique, cette dernière peut être estérifiée, réduite en aldéhyde, réduite en alcool puis éthérifiée, amidifiée, nitrilée ou autres. Toutes les fonctions peuvent être obtenues selon des techniques bien connues de l'homme de métier, en fonction de l'utilisation finale que l'on destine au dithiocarbamate.

Ainsi, le dithiocarbamate cyclique fonctionnalisé de formule (I) obtenu à l'issue de l'étape d/ ou e/ peut être soumis à une ou plusieurs réactions chimiques supplémentaires pour obtenir un ou plusieurs dithiocarbamate avec des fonctions différentes, les dites réactions chimiques étant toutes réactions connues de l'homme du métier.

Selon un mode de réalisation de l'invention, on met en présence un dérivé de L-sérine tel que l'O-acétyl-L-sérine, une enzyme telle que l'O-acétyl-L-sérine sulfhydrylase, un cofacteur, tel que le pyridoxal-5'-phosphate (PLP), et du trithiocarbonate basique, tel que le trithiocarbonate de sodium, il s'avère de façon surprenante qu'un des produits principaux obtenu est un dithiocarbamate cyclique répondant au nom d'acide L-raphanusamique, l'acide raphanusamique étant représenté par la formule :

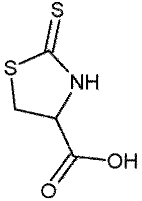

Selon un autre mode de réalisation de l'invention, on met en présence un dérivé de _{L}-homosérine tel que l'O-acétyl-L-homosérine, une enzyme telle que l'O-acétyl-_{L}-homosérine sulfhydrylase, un cofacteur, tel que le pyridoxal-5'-phosphate (PLP), et du trithiocarbonate basique, tel que le trithiocarbonate de sodium. Il s'avère de façon surprenante que l'un des produits principaux obtenu est un dithiocarbamate cyclique répondant au nom d'acide L-homoraphanusamique (homologue supérieur cyclique de l'acide L-raphanusamique), l'acide homoraphanusamique étant représenté par la formule :

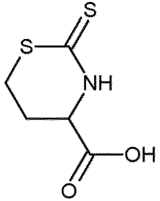

Il a été observé que la synthèse du dithiocarbamate peut s'accompagner de la production d'un mercaptan de formule (III) : HS-CH₂(CH₂)ₙ-C*H(NHR₁)-X-R₂ dans laquelle n, R₁, R₂, X et * sont tels que définis précédemment. Ce mercaptan peut avantageusement servir de matière première pour la synthèse du dithiocarbamate par réaction avec du disulfure de carbone en milieu basique.

Par exemple, dans le cas de la synthèse de l'acide homoraphanusamique, le mercaptan produit est l'homocystéine qui réagit avec du disulfure de carbone selon le schéma suivant :

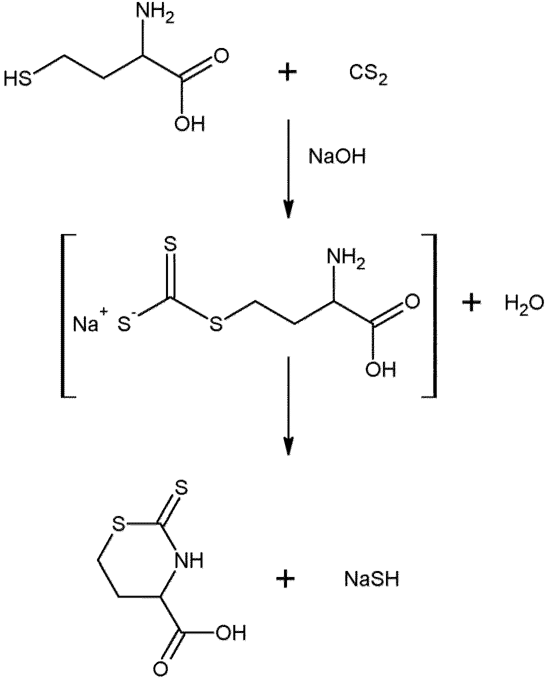

Le composé entre crochets est un composé intermédiaire qui apparaît au cours du procédé. Ce composé, ainsi que ses autres sels d'alcalin, d'alcalino-terreux ou d'ammonium, sont dénommés dans la suite « trithiocabonates » (« trithiocarbonate sodique » dans le cas ou le contre-ion est l'ion sodium) de l'homocystéine.

De manière plus générale, le composé intermédiaire peut être un composé de formule (IV) :

X₂⁺S⁻-C=(S)-S-CH₂-(CH₂)ₙC*H(NHR₁)-X-R₂ (IV)

dans laquelle R₁, R₂, X, * et n sont tels que définis précédemment et X₂ représente un alcalin, un alcalino-terreux, ou un groupement ammonium, de préférence Na, K, NH₄ ou Ca, de préférence encore Na.

Selon un autre mode de réalisation préféré de l'invention, du disulfure de carbone peut être ajouté en continu ou en batch au cours de la réaction.

L'ajout de disulfure de carbone permet notamment d'augmenter le rendement de synthèse du dithiocarbamate.

Selon un mode de réalisation préféré de l'invention, le dérivé de la L-sérine est l'O-acétyl-L-sérine, le trithiocarbonate est le trithiocarbonate de sodium et l'enzyme utilisée est l'O-acétyl-_{L}-sérine sulfhydrylase.

Selon un mode de réalisation préféré de l'invention, le dithiocarbamate cyclique fonctionnalisé de formule (I) obtenu selon le procédé est l'acide L-raphanusamique.

Selon un autre mode de réalisation préféré de l'invention, le dérivé de la _{L}-homosérine est l'O-acétyl-L-homosérine, le trithiocarbonate est le trithiocarbonate de sodium et l'enzyme utilisée est la O-acétyl-L-homosérine sulfhydrylase.

Selon un mode de réalisation préféré de l'invention, le dithiocarbamate cyclique fonctionnalisé de formule (I) obtenu selon le procédé est l'acide _{L}-homoraphanusamique.

Comme indiqué précédemment, la configuration du carbone asymétrique est conservée tout au long de la réaction. Ceci permet d'obtenir un énantiomère particulier ce qui peut présenter un avantage pour certaines applications, notamment dans le domaine médical ou pharmaceutique.

De plus, l'acide carboxylique présent sur le dithiocarbamate fonctionnalisé de formule (I) peut permettre « d'accrocher » une large diversité de composés ou molécules rendant possible un greffage sur support organique ou inorganique.

Les dithiocarbamates cycliques fonctionnalisés de formule (I) préparés selon le procédé selon l'invention peuvent être utilisés en tant que précurseurs de radicaux, intermédiaires de synthèse organique, agents de vulcanisation, chélateurs ou inhibiteurs d'enzyme. Leurs domaines d'application sont divers et ils peuvent par exemple entrer dans la composition de fongicides, de désherbants, de pesticides ou d'insecticides dans le domaine agricole, être utilisés dans l'industrie du caoutchouc ou encore dans l'industrie pharmaceutique dans le traitement de maladies telles que le cancer ou le VIH.

### EXEMPLES

### Exemple 1 : Synthèse enzymatique de l'acide L-homoraphanusamique

### Étape 1 :

L'O-acétyl-L-homosérine (OAHS) a été synthétisée à partir de _{L}-homosérine et d'anhydride acétique selon Sadamu Nagai, « Synthesis of O-acetyl-L-homoserine », Academic Press, (1971), vol.17, pp. 423-424.

### Étape 2 :

Dans un réacteur thermostaté en verre de 250 mL, sont introduits 10 g (62 mmol) d'OAHS, précédemment synthétisés, dans 140 mL d'eau distillée. La solution est portée à 35°C sous agitation mécanique. Le pH du milieu réactionnel est de 4,8. Avant d'ajouter l'enzyme, le pH est fixé à 6,5 par quelques gouttes de la solution de trithiocarbonate de sodium (4,78 g ; 31 mmol, dissous dans 20 mL d'eau distillée). On effectue un prélèvement (à t = 0) de 1 mL du milieu réactionnel. Une solution de pyridoxal-5'-phosphate (10 mmol, 0,4 g) et l'enzyme, l'O-acétyl-L-homosérine sulfhydrylase, (0,6 g) sont dissous dans 10 mL d'eau puis ajoutés dans le réacteur.

La réaction commence, ce qui engendre une diminution du pH. Le milieu réactionnel est maintenu à un pH de 6,5 par ajout de trithiocarbonate de sodium lentement via l'ampoule de coulée. Des prélèvements (1 mL) sont effectués au cours de la réaction. Les analyses par potentiométrie, CCM, HPLC et UPLC/UV-masse montrent une disparition progressive des réactifs (OAHS et Na₂CS₃) et l'apparition progressive, en quantités de plus en plus importantes, du composé suivant :

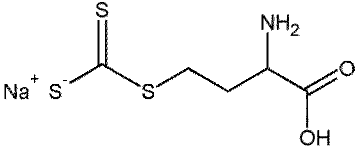

Ce composé intermédiaire disparaît à son tour progressivement pour donner en quantité équimolaires :
- l'acide L-homoraphanusamique (le dithiocarbamate cyclique fonctionnalisé) 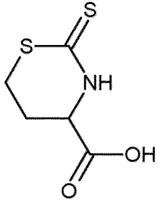
- et la L-homocystéine

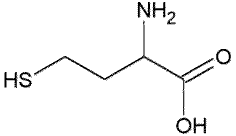

Les seuls autres produits observés après la disparition totale de l'OAHS sont des traces d'homosérine (hydrolyse de l'OAHS).

### Étape 3 : Séparation et isolation du dithiocarbamate :

Le milieu réactionnel est concentré par évaporation partielle de l'eau (de façon à éviter la précipitation de l'acétate de sodium présent dans le milieu réactionnel) sous pression réduite à 30°C. Un précipité se forme car le dithiocarbamate s'avère être le moins soluble des composés présents dans le milieu réactionnel. Après filtration et séchage, on obtient 4,9 g de dithiocarbamate. Le rendement isolé global de dithiocarbamate est de 45% (4,9 g obtenus sur 11 g théoriquement attendus). Des analyses complémentaires sur ce produit sec ont montré que ce solide ne contient que des traces d'homocystéine.

### Exemple 2 : Synthèse de dithiocarbamate (sans enzyme, ni coenzyme)

L'exemple 1 a été répété à la seule différence que la solution de pyridoxal-5'-phosphate (10 mmol ; 0,4 g) et l'enzyme, l'O-acétyl-L-homosérine sulfhydrylase (0,6 g) dissout dans 10 mL d'eau n'ont pas été ajoutées dans le réacteur. Il s'avère que la réaction ne démarre pas et qu'il n'est pas possible d'ajouter continuellement la solution de trithiocarbonate en cherchant à conserver un pH de 6,5. En augmentant à pH 8, puis à pH 12, par ajout de solution de trithiocarbonate de sodium, la seule réaction observée est un début d'hydrolyse de l'OAHS en homoserine. Cet exemple montre que la synthèse de dithiocarbamate doit être catalysée par une enzyme pour être performante.

### Exemple 3 : Synthèse enzymatique du dithiocarbamate (avec ajout de CS₂ en fin de réaction)

### Étape 1 :

La synthèse de l'O-acétyl-L-homoserine (OAHS) à partir de _{L}-homosérine a été réalisée selon un protocole tiré de la littérature (Sadamu Nagai, « Synthesis of O-acetyl-homoserine », Academic Press, (1971), vol.17, pp. 423-424).

### Étape 2 :

Dans un réacteur thermostaté en verre de 250 mL, sont introduits 10 g (62 mmol) d'OAHS dans 140 mL d'eau distillée. La solution est portée à 35°C sous agitation mécanique. Le pH du milieu réactionnel est de 4,8. Avant d'ajouter l'enzyme, le pH est fixé à 6,5 par ajout de quelques gouttes de la solution de trithiocarbonate de sodium (la quantité totale ajoutée tout au long de la réaction est égale à 4,78 g, soit 31 mmol, dissous dans 20 mL d'eau distillée). On effectue un prélèvement (à t = 0) d'1 mL du milieu réactionnel.

On prépare une solution de 10 mL d'eau distillée contenant 400 µL d'une solution de pyridoxal-5'-phosphate (10 mmol/L) et de 0,6 g d'enzyme (O-acétyl-L-homosérine sulfhydrylase). Une diminution du pH indiquant la formation d'acide acétique permet de dire que la réaction commence. Il est nécessaire de maintenir le milieu réactionnel à un pH égal à 6,5. Pour cela, la solution de trithiocarbonate de sodium est ajoutée lentement via l'ampoule de coulée. Des prélèvements (1 mL) sont effectués au cours de la réaction.

Lorsque les analyses par potentiométrie indiquent une conversion de 50% de l'OAHS en homocystéine, 1,87 mL de disulfure de carbone (31 mmol) sont ajoutés au milieu réactionnel. Le pH du milieu réactionnel est ajusté à 10 avec une solution d'hydroxyde de sodium 1M. Le milieu réactionnel est alors porté à 50°C. Une disparition de la cystéine par analyse potentiomètrique est observée. Une solution d'acide chlorhydrique (2N) est ensuite utilisée pour abaisser à 5 le pH du milieu réactionnel.

Les analyses complémentaires par CCM, HPLC et UPLC/UV-masse montrent la formation d'un produit principal l'acide L-homoraphanusamique.

Les seuls autres produits observés après la disparition totale de l'OAHS sont des traces d'homoserine (hydrolyse de l'OAHS) ainsi que des traces d'homocystéine.

### Étape 3 : Séparation et isolation du dithiocarbamate

Le milieu réactionnel est concentré par évaporation partielle de l'eau (de façon à éviter la précipitation de l'acétate de sodium et des autres sels présents dans le milieu réactionnel) sous pression réduite à 30°C. Ainsi un précipité se forme car le dithiocarbamate s'avère être l'espèce la moins soluble dans l'eau. Après filtration et séchage, on obtient 9,2 g de dithiocarbamate. Le rendement isolé global de dithiocarbamate est de 9,2 g sur 11 g théoriques soit 84%.

### Exemple 4 : Synthèse enzymatique du dithiocarbamate (avec ajout de CS₂ pendant la réaction)

### Étape 1 :

La synthèse de l'O-acétyl-L-homosérine (OAHS) à partir de _{L}-homosérine a été réalisée selon un protocole tiré de la littérature (source : « Sadamu Nagai, Synthesis of O-acetyl-homoserine », Academic Press, (1971), vol.17, pp. 423-424).

### Étape 2 :

Dans un réacteur thermostaté en verre de 250 mL, sont introduits 10 g (61 mmol) d'OAHS précédemment synthétisés dans 140 mL d'eau distillée. La solution est portée à 35°C sous agitation mécanique. Le pH du milieu réactionnel est de 4,8. Avant de mettre l'enzyme, le pH est fixé 7,2 par ajout de quelques gouttes de la solution de trithiocarbonate et de disulfure de carbone (4,78 g de trithiocarbonate, 31 mmol, 1,87 mL de disulfure de carbone ; 31 mmol dissous dans 20 mL d'eau distillé).

On effectue un prélèvement (à t = 0) d'1 mL du milieu réactionnel. On prépare une solution de 10 mL d'eau distillé contenant 400 µL d'une solution de pyridoxal-5'-phosphate (10 mmol/L) et de 0.6 g d'enzyme (O-acétyl-L-homosérine sulfhydrylase). Une diminution du pH indiquant la formation d'acide acétique permet de dire que la réaction commence. Il est nécessaire de maintenir le milieu réactionnel à un pH égal à 7,2. Pour cela la solution de trithiocarbonate de sodium est ajoutée lentement via l'ampoule de coulée. Des prélèvements (1 mL) sont effectués au cours de la réaction. Les analyses par potentiométrie, CCM, HPLC et UPLC/UV-masse (après dérivatisation) montrent une disparition progressive des réactifs (OAHS et Na₂CS₃) et l'apparition progressive en quantité de plus en plus importante de l'acide _{L}-homoraphanusamique. Lorsque la totalité de l'OAHS a réagi, le pH du milieu est descendu à 5 à l'aide d'une solution d'acide chlorhydrique 2M.

On obtient l'acide L-homoraphanusamique (le dithiocarbamate).

La dérivatisation pour la méthode UPLC/UV-Masse a été faite par la même méthode décrite dans l'exemple 1.

Les seuls autres produits observés après la disparition totale de l'OAHS sont des traces d'homoserine (hydrolyse de l'OAHS) ainsi que des traces d'homocystéine.

### Étape 3 : Séparation et isolation du dithiocarbamate

Le milieu réactionnel est concentré par évaporation partielle de l'eau (de façon à éviter la précipitation de l'acétate de sodium et des autres sels présents dans le milieu réactionnel) sous pression réduite à 30°C. Ainsi un précipité se forme car le dithiocarbamate s'avère être l'espèce la moins soluble dans l'eau. Après filtration et séchage, on obtient 8,3 g de dithiocarbamate. Le rendement isolé global de dithiocarbamate est 8,3 g sur 11 g théoriques soit 75,4%.

## Revendications

1. Procédé de synthèse d'un dithiocarbamate cyclique fonctionnalisé de formule (I) : 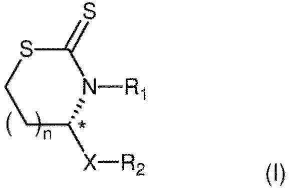 dans laquelle
- R₁ est un hydrogène ou une chaîne hydrocarbonée ramifiée ou non, saturée ou insaturée, linéaire ou cyclique, aromatique ou non, comportant de 1 à 20 atomes de carbone, et pouvant comporter un ou plusieurs hétéroatomes choisis parmi O, S, N, P et Si ;
- X représente -C(=O)- ou -CH₂- ou -CN ;
- R₂ est (i) soit nul (quand X représente -CN), (ii) soit un hydrogène, (iii) soit -OR₃, R₃ étant un hydrogène ou une chaîne hydrocarbonée ramifiée ou non, saturée ou insaturée, linéaire ou cyclique, aromatique ou non, comportant de 1 à 20 atomes de carbone, et pouvant comporter ou plusieurs hétéroatomes choisis parmi O, S, N, P et Si, (iv) soit -NR₄R₅ avec R₄ et R₅, différents ou non, étant un hydrogène ou une chaîne hydrocarbonée ramifiée ou non, saturée ou insaturée, linéaire ou cyclique, aromatique ou non, comportant de 1 à 20 atomes de carbone, et pouvant comporter un ou plusieurs hétéroatomes choisis parmi O, S, N, P et Si ;
- n est égal à 0, 1 ou 2, de préférence 1 ; et
- * représente un carbone asymétrique,
ledit procédé comprenant les étapes de :
a/ fourniture d'au moins un composé de formule (II) :
G-(CH₂)ₙ-C*H(NHR₁)-X-R₂ (II)
dans laquelle
- n, R₁, R₂, X, et * sont tels que définis précédemment,
- G représente soit (i) R₆-C(=O)-O-CH₂-, soit (ii) (R₇O)(R₈O)-P(=O)-O-CH₂-, soit (iii) R₇O-SO₂-O-CH₂- ;
- R₆ est un hydrogène ou une chaîne hydrocarbonée ramifiée ou non, saturée ou insaturée, linéaire ou cyclique, aromatique ou non, comportant de 1 à 20 atomes de carbone, et pouvant comporter un ou plusieurs hétéroatomes choisis parmi O, S, N, P et Si ;
- R₇ et R₈, identiques ou différents, sont choisis indépendamment l'un de l'autre parmi un proton H, un alcalin, un alcalino-terreux ou un ammonium, de préférence un proton H ou un alcalin, et plus particulièrement un proton H ou Na,
b/ fourniture d'au moins un trithiocarbonate inorganique,
c/ réaction entre ledit au moins composé de formule (II) et ledit au moins trithiocarbonate inorganique en présence d'au moins une enzyme choisie parmi les sulfhydrylases, et de préférence une sulfhydrylase associée audit composé de formule (II),
d/ obtention d'au moins un dithiocarbamate cyclique fonctionnalisé de formule (I),
e/ séparation et isolation dudit au moins un dithiocarbamate cyclique fonctionnalisé de formule (I),
f/ de façon optionnelle, fonctionnalisation supplémentaire du dithiocarbamate cyclique fonctionnalisé de formule (I) obtenu à l'étape d/ ou e/,
les étapes a/ et b/ étant, ou non, effectuées de manière simultanée.

2. Procédé selon la revendication 1 dans lequel le dithiocarbamate cyclique fonctionnalisé de formule (I) est énantiomériquement pur.

3. Procédé selon l'une des revendications précédentes dans lequel le dithiocarbamate cyclique fonctionnalisé de formule (I) est l'acide L-raphanusamique ou l'acide _{L}-homoraphanusamique.

4. Procédé selon l'une des revendications précédentes dans lequel le composé de formule (II) est choisi parmi les dérivés de la L-sérine et les dérivés de la _{L}-homosérine.

5. Procédé selon la revendication 4 dans lequel le dérivé de la L-sérine est choisi parmi l'O-phospho-_{L}-sérine, l'O-succinyl-L-sérine, l'O-acétyl-L-sérine, l'O-acétoacétyl-_{L}-sérine, la O-propio-L-sérine, la O-coumaroyl-L-sérine, la O-malonyl-L-sérine, l'O-hydroxyméthylglutaryl-L-sérine, la O-pimélyl-L-sérine et la O-sulfato-L-sérine, de préférence l'O-phospho-_{L}-sérine, l'O-succinyl-L-sérine, l'O-acétyl-_{L}-sérine et la O-sulfato-_{L}-sérine, et plus particulièrement l'O-acétyl-L-sérine.

6. Procédé selon la revendication 4 dans lequel le dérivé de la L-homosérine est choisi parmi l'O-succinyl-L-homosérine, l'O-acétyl-L-homosérine, l'O-acétoacétyl-_{L}-homosérine, l'O-propio-L-homosérine, l'O-coumaroyl-L-homosérine, l'O-malonyl-_{L}-homosérine, l'O-hydroxyméthylglutaryl-L-homosérine, l'O-pimélyl-L-homosérine, l'O-phospho-L-homosérine et l'O-sulfato-L-homosérine, de préférence l'O-succinyl-_{L}-homosérine, l'O-acétyl-L-homosérine, l'O-phospho-L-homosérine et l'O-sulfato-_{L}-homosérine, et plus particulièrement l'O-acétyl-L-homosérine.

7. Procédé selon l'une des revendications précédentes dans lequel la sulfhydrylase est choisie parmi les sulfhydrylases associées aux dérivés de la L-sérine et les sulfhydrylases associées aux dérivés de la _{L}-homosérine.

8. Procédé selon la revendication 7 dans lequel la sulfhydrylase associée au dérivé de la L-sérine est choisie parmi l'O-phospho-_{L}-sérine sulfhydrylase, l'O-succinyl-L-sérine sulfhydrylase, l'O-acétyl-_{L}-sérine sulfhydrylase, l'O-acétoacétyl-L-sérine sulfhydrylase, l'O-propio-L-sérine sulfhydrylase, l'O-coumaroyl-L-sérine sulfhydrylase, l'O-malonyl-L-sérine sulfhydrylase, l'O-hydroxyméthylglutaryl-L-sérine sulfhydrylase, l'O-pimélyl-L-sérine sulfhydrylase et l'O-sulfato-L-sérine sulfhydrylase, de préférence l'O-phospho-_{L}-sérine sulfhydrylase, l'O-succinyl-L-sérine sulfhydrylase, l'O-acétyl-_{L}-sérine sulfhydrylase et l'O-sulfato-L-sérine sulfhydrylase, et plus particulièrement l'O-acétyl-_{L}-sérine sulfhydrylase.

9. Procédé selon la revendication 7 dans lequel la sulfhydrylase associée au dérivé de la _{L}-homosérine est choisie parmi l'O-phospho-L-homosérine sulfhydrylase, l'O-succinyl-_{L}-homosérine sulfhydrylase, l'O-acétyl-_{L}-homosérine sulfhydrylase, l'O-acétoacétyl-_{L}-homosérine sulfhydrylase, l'O-propio-L-homosérine sulfhydrylase, l'O-coumaroyl-_{L}-homosérine sulfhydrylase, l'O-malonyl-L-homosérine sulfhydrylase, l'O-hydroxyméthyl-glutaryl-L-homosérine sulfhydrylase, l'O-pimélyl-L-homosérine sulfhydrylase et l'O-sulfato-_{L}-homosérine sulfhydrylase, de préférence l'O-phospho-L-homosérine sulfhydrylase, l'O-succinyl-L-homosérine sulfhydrylase, l'O-acétyl-_{L}-homosérine sulfhydrylase et l'O-sulfato-L-homosérine sulfhydrylase, et plus particulièrement l'O-acétyl-_{L}-homosérine sulfhydrylase.

10. Procédé selon l'une quelconque des revendications précédentes dans lequel le trithiocarbonate inorganique est choisi parmi un trithiocarbonate d'alcalin, un trithiocarbonate d'alcalino-terreux ou un trithiocarbonate d'ammonium, de préférence le trithiocarbonate de sodium, le trithiocarbonate de potassium, le trithiocarbonate de calcium et le trithiocarbonate d'ammonium, et plus particulièrement le trithiocarbonate de sodium.

11. Procédé selon l'une quelconque des revendications précédentes comportant une étape f/ optionnelle de fonctionnalisation supplémentaire du dithiocarbamate cyclique fonctionnalisé de formule (I) obtenu à l'étape d/ ou à l'étape e/.

12. Acide L-homoraphanusamique préparé selon le procédé décrit dans les revendications 1 à 11.

## Patentansprüche

1. Syntheseverfahren eines funktionalisierten cyclischen Dithiocarbamats von Formel (I): 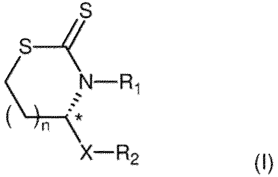 wobei
- R₁ Wasserstoff oder eine verzweigte oder unverzweigte, gesättigte oder ungesättigte, lineare oder cyclische, aromatische oder nicht-aromatische Kohlenwasserstoffkette ist, umfassend 1 bis 20 Kohlenstoffatome, und ein oder mehrere Heteroatome umfassen kann, ausgewählt aus O, S, N, P und Si;
- X -C(=O)- oder -CH₂- oder -CN darstellt;
- R₂ (i) entweder Null ist (wenn X -CN darstellt), (ii) oder Wasserstoff ist, (iii) oder
- OR₃ ist, wobei R₃ Wasserstoff oder eine verzweigte oder unverzweigte, gesättigte oder ungesättigte, lineare oder cyclische, aromatische oder nicht-aromatische Kohlenwasserstoffkette ist, umfassend 1 bis 20 Kohlenstoffatome, und ein oder mehrere Heteroatome umfassen kann, ausgewählt aus O, S, N, P und Si, (iv) oder - NR₄R₅ ist, wobei R₄ und R₅, verschieden sind oder nicht, Wasserstoff oder eine verzweigte oder unverzweigte, gesättigte oder ungesättigte, lineare oder cyclische, aromatische oder nicht-aromatische Kohlenwasserstoffkette sind, umfassend 1 bis 20 Kohlenstoffatome, und ein oder mehrere Heteroatome umfassen können, ausgewählt aus O, S, N, P und Si;
- n 0, 1 oder 2, vorzugsweise 1, ist; und
- * einen asymmetrischen Kohlenstoff darstellt, das Verfahren umfassend die folgenden Schritte:
a) Bereitstellung mindestens einer Verbindung von Formel (II):
G-(CH₂)ₙ-C*H(NHR₁)-X-R₂ (II)
wobei
- n, R₁, R₂, X und * wie vorstehend definiert sind,
- G entweder (i) R₆-C(=O)-O-CH₂-, oder (ii) (R₇O)(R₈O)-P(=O)-O-CH₂- oder (iii) R₇O-SO₂-O-CH₂- darstellt;
- R₆ Wasserstoff oder eine verzweigte oder unverzweigte, gesättigte oder ungesättigte, lineare oder cyclische, aromatische oder nicht-aromatische Kohlenwasserstoffkette ist, umfassend 1 bis 20 Kohlenstoffatome, und ein oder mehrere Heteroatome umfassen kann, ausgewählt aus O, S, N, P und Si;
- R₇ und R₈, die gleich oder verschieden sind, unabhängig ausgewählt sind aus einem H-Proton, einem Alkali, einem Erdalkali oder einem Ammonium, vorzugsweise einem H-Proton oder einem Alkali und bevorzugter ein H- oder Na-Proton,
b) Bereitstellung von mindestens einem anorganischen Trithiocarbonat,
c) Reaktion zwischen der mindestens einen Verbindung von Formel (II) und dem mindestens einen anorganischen Trithiocarbonat in Anwesenheit von mindestens einem Enzym, das ausgewählt ist aus Sulfhydrylasen, und vorzugsweise einer Sulfhydrylase, die mit der Verbindung von Formel (II) assoziiert ist,
d) Erlangen von mindestens einem funktionalisierten cyclischen Dithiocarbamat von Formel (I),
e) Abtrennen und Isolieren des mindestens einen funktionalisierten cyclischen Dithiocarbamats von Formel (I),
f) optional, ergänzendes Funktionalisieren des funktionalisierten cyclischen Dithiocarbamats von Formel (I), das in Schritt d) oder e) erlangt wird,
wobei die Schritte a) und b) gleichzeitig durchgeführt werden oder nicht.

2. Verfahren nach Anspruch 1, bei dem das funktionalisierte cyclische Dithiocarbamat von Formel (I) enantiomerisch gesehen rein ist.

3. Verfahren nach einem der vorherigen Ansprüche, wobei das funktionalisierte cyclische Dithiocarbamat von Formel (I) L-Raphanusaminsäure oder L-Homoraphanusaminsäure ist.

4. Verfahren nach einem der vorherigen Ansprüche, wobei die Verbindung von Formel (I) ausgewählt ist aus L-Serin-Derivaten und L-Homoserin-Derivaten.

5. Verfahren nach Anspruch 4, wobei das L-Serin-Derivat ausgewählt ist aus O-Phospho-L-serin, O-Succinyl-L-serin, O-Acetyl-L-serin, O-Acetoacetyl-L-serin, O-Propio-L-serin, O-Coumaroyl-L-serin, O-Malonyl-L-serin, o-Hydroxymethylglutaryl-L-serin, O-Pimelyl-L-serin und O-Sulfato-L-serin, vorzugsweise O-Phospho-L-serin, O-Succinyl-L-serin, O-Acetyl-L-serin und O-Sulfato-L-serin, und bevorzugter O-Acetyl-L-serin.

6. Verfahren nach Anspruch 4, wobei das L-Homoserin-Derivat ausgewählt ist aus O-Succinyl-L-homoserin, O-Acetyl-L-homoserin, O-Acetoacetyl-L-homoserin, O-Propio-L-homoserin, O-Coumaroyl-L-homoserin, O-Malonyl- L-homoserin, O-Hydroxymethylglutaryl-L-homoserin, O-Pimelyl-L-homoserin, O-Phospho-L-homoserin und O-Sulfato-L-homoserin, vorzugsweise O-Succinyl-L-homoserin, O-Acetyl-L-homoserin, O-Phospho-L-homoserin und O-Sulfato-L-homoserin und insbesondere O-Acetyl-L-homoserin.

7. Verfahren nach einem der vorherigen Ansprüche, wobei die Sulfhydrylase ausgewählt ist aus Sulfhydrylasen, die mit L-Serin-Derivaten assoziiert sind, und Sulfhydrylasen, die mit L-Homoserin-Derivaten assoziiert sind.

8. Verfahren nach Anspruch 7, wobei die mit L-Serin-Derivat assoziierte Sulfhydrylase ausgewählt ist aus O-Phospho-L-serin-sulfhydrylase, O-Succinyl-L-serin-sulfhydrylase, O-Acetyl-L-serin-sulfhydrylase, O-Acetoacetyl-L-serin-sulfhydrylase, O-Propio-L-serin-sulfhydrylase, O-Coumaroyl-L-serin-sulfhydrylase, O-Malonyl-L-serin-sulfhydrylase, o-Hydroxymethylglutaryl-L-serin-sulfhydrylase, O-Pimelyl-L-serin-sulfhydrylase und O-Sulfato-L-serin-sulfhydrylase, vorzugsweise O-Phospho-L-serin-sulfhydrylase, O-Succinyl-L-serin-sulfhydrylase, O-Acetyl-L-serin-sulfhydrylase und O-Sulfato-L-serin-sulfhydrylase und insbesondere O-Acetyl-L-serin-sulfhydrylase.

9. Verfahren nach Anspruch 7, wobei die mit L-Homoserin-Derivat assoziierte Sulfhydrylase ausgewählt ist aus O-Phospho-L-homoserin-sulfhydrylase, O-Succinyl-L-homoserin-sulfhydrylase, o-Acetyl-L-homoserin-sulfhydrylase, O-Acetoacetyl L-homoserin-sulfhydrylase, O-Propio-L-homoserin-sulfhydrylase, O-Cumaroyl L-homoserin-sulfhydrylase, O-Malonyl-L-homoserin-sulfhydrylase, o-Hydroxymethylglutaryl-L-homoserin-sulfhydrylase, O-Pimelyl-L-homoserin-sulfhydrylase und O-Sulfato L-homoserin-sulfhydrylase, vorzugsweise O-Phospho-L-homoserin-sulfhydrylase, o-Succinyl-L-homoserin-sulfhydrylase, O-Acetyl-L-homoserin-sulfhydrylase und O-Sulfato-L-homoserin-sulfhydrylase, und insbesondere O-Acetyl-L-homoserin-sulfhydrylase.

10. Verfahren nach einem der vorherigen Ansprüche, wobei das anorganische Trithiocarbonat ausgewählt ist aus Alkalitrithiocarbonat, Erdalkalitrithiocarbonat oder Ammoniumtrithiocarbonat, vorzugsweise Natriumtrithiocarbonat, Kaliumtrithiocarbonat, Calciumtrithiocarbonat und Ammoniumtrithiocarbonat, und insbesondere Natriumtrithiocarbonat.

11. Verfahren nach einem der vorherigen Ansprüche, umfassend einen optionalen Schritt f) einer ergänzenden Funktionalisierung des funktionalisierten cyclischen Dithiocarbamats von Formel (I), das in Schritt d) oder Schritt e) erlangt wird.

12. L-Homoraphanusamidsäure, hergestellt nach dem in den Ansprüchen 1 bis 11 beschriebenen Verfahren.

## Claims

1. A process for synthesizing a functionalized cyclic dithiocarbamate of formula (I): 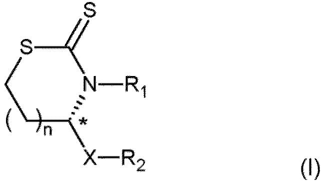 in which
- R₁ is a hydrogen or an aromatic or nonaromatic, linear or cyclic, saturated or unsaturated, branched or unbranched, hydrocarbon-based chain including from 1 to 20 carbon atoms, and which may include one or more heteroatoms chosen from O, S, N, P and Si;
- X represents -C(=O)- or -CH₂- or -CN;
- R₂ is (i) either nonexistent (when X represents -CN), (ii) or a hydrogen, (iii) or -OR₃, R₃ being a hydrogen or an aromatic or nonaromatic, linear or cyclic, saturated or unsaturated, branched or unbranched, hydrocarbon-based chain including from 1 to 20 carbon atoms, and which may comprise or more heteroatoms chosen from O, S, N, P and Si, (iv) or - NR₄R₅, with R₄ and R₅, which are or are not different, being a hydrogen or an aromatic or nonaromatic, linear or cyclic, saturated or unsaturated, branched or unbranched, hydrocarbon-based chain including from 1 to 20 carbon atoms, and which may include one or more heteroatoms chosen from O, S, N, P and Si;
- n is equal to 0, 1 or 2, preferably 1; and
- * represents an asymmetric carbon;
said process comprising the steps of:
a/ providing at least one compound of formula (II):
G-(CH₂)ₙ-C*H(NHR₁)-X-R₂ (II)
in which
- n, R₁, R₂, X and * are as defined previously,
- G represents either (i) R₆-C(=O)-O-CH₂-, or (ii) (R₇O)(R₈O)-P(=O)-O-CH₂-, or (iii) R₇O-SO₂-O-CH₂-;
- R₆ is a hydrogen or an aromatic or nonaromatic, linear or cyclic, saturated or unsaturated, branched or unbranched, hydrocarbon-based chain including from 1 to 20 carbon atoms, and which may include one or more heteroatoms chosen from O, S, N, P and Si;
- R₇ and R₈, which may be identical or different, are chosen, independently of each other, from a proton H, an alkali metal, an alkaline-earth metal or an ammonium, preferably a proton H or an alkali metal and more particularly a proton H or Na;
b/ providing at least one inorganic trithiocarbonate;
c/ reaction between said at least compound of formula (II) and said at least inorganic trithiocarbonate in the presence of at least one enzyme chosen from sulfhydrylases, and preferably a sulfhydrylase associated with said compound of formula (II);
d/ production of at least one functionalized cyclic dithiocarbamate of formula (I);
e/ separation and isolation of said at least one functionalized cyclic dithiocarbamate of formula (I);
f/ optionally, additional functionalization of the functionalized cyclic dithiocarbamate of formula (I) obtained in step d/ or e/;
steps a/ and b/ optionally being performed simultaneously.

2. The process as claimed in claim 1, in which the functionalized cyclic dithiocarbamate of formula (I) is enantiomerically pure.

3. The process as claimed in either of the preceding claims, in which the functionalized cyclic dithiocarbamate of formula (I) is L-raphanusamic acid or L-homoraphanusamic acid.

4. The process as claimed in one of the preceding claims, in which the compound of formula (II) is chosen from L-serine derivatives and L-homoserine derivatives.

5. The process as claimed in claim 4, in which the L-serine derivative is chosen from O-phospho-L-serine, O-succinyl-L-serine, O-acetyl-L-serine, O-acetoacetyl-L-serine, O-propio-L-serine, O-coumaroyl-L-serine, O-malonyl-L-serine, O-hydroxymethylglutaryl-L-serine, O-pimelyl-L-serine and O-sulfato-L-serine, preferably O-phospho-L-serine, O-succinyl-L-serine, O-acetyl-L-serine and O-sulfato-L-serine, and more particularly O-acetyl-L-serine.

6. The process as claimed in claim 4, in which the L-homoserine derivative is chosen from O-succinyl-L-homoserine, O-acetyl-L-homoserine, O-acetoacetyl-L-homoserine, propio-L-homoserine, O-coumaroyl-L-homoserine, O-malonyl-L-homoserine, O-hydroxymethylglutaryl-L-homoserine, O-pimelyl-L-homoserine, O-phospho-L-homoserine and O-sulfato-L-homoserine, preferably O-succinyl-L-homoserine, O-acetyl-L-homoserine, O-phospho-homoserine, and O-sulfato-L-homoserine, and more particularly O-acetyl-L-homoserine.

7. The process as claimed in one of the preceding claims, in which the sulfhydrylase is chosen from the sulfhydrylases associated with the L-serine derivatives and the sulfhydrylases associated with the L-homoserine derivatives.

8. The process as claimed in claim 7, in which the sulfhydrylase associated with the L-serine derivative is chosen from O-phospho-L-serine sulfhydrylase, O-succinyl-L-serine sulfhydrylase, O-acetyl-L-serine sulfhydrylase, O-acetoacetyl-L-serine sulfhydrylase, O-propio-L-serine sulfhydrylase, O-coumaroyl-L-serine sulfhydrylase, O-malonyl-L-serine sulfhydrylase, O-hydroxymethylglutaryl-L-serine sulfhydrylase, O-pimelyl-L-serine sulfhydrylase and O-sulfato-L-serine sulfhydrylase, preferably O-phospho-L-serine sulfhydrylase, O-succinyl-L-serine sulfhydrylase, O-acetyl-L-serine sulfhydrylase and O-sulfato-L-serine sulfhydrylase, and more particularly O-acetyl-L-serine sulfhydrylase.

9. The process as claimed in claim 7, in which the sulfhydrylase associated with the L-homoserine derivative is chosen from O-phospho-L-homoserine sulfhydrylase, O-succinyl-L-homoserine sulfhydrylase, O-acetyl-L-homoserine sulfhydrylase, O-acetoacetyl-L-homoserine sulfhydrylase, O-propio-L-homoserine sulfhydrylase, O-coumaroyl-L-homoserine sulfhydrylase, O-malonyl-L-homoserine sulfhydrylase, O-hydroxymethylglutaryl-L-homoserine sulfhydrylase, O-pimelyl-L-homoserine sulfhydrylase and O-sulfato-L-homoserine sulfhydrylase, preferably O-phospho-homoserine sulfhydrylase, O-succinyl-L-homoserine sulfhydrylase, O-acetyl-L-homoserine sulfhydrylase and O-sulfato-L-homoserine sulfhydrylase, and more particularly O-acetyl-L-homoserine sulfhydrylase.

10. The process as claimed in any one of the preceding claims, in which the inorganic trithiocarbonate is chosen from an alkali metal trithiocarbonate, an alkaline-earth metal trithiocarbonate and an ammonium trithiocarbonate, preferably sodium trithiocarbonate, potassium trithiocarbonate, calcium trithiocarbonate and ammonium trithiocarbonate, and more particularly sodium trithiocarbonate.

11. The process as claimed in any one of the preceding claims, including an optional step f/ of additional functionalization of the functionalized cyclic dithiocarbamate of formula (I) obtained in step d/ or in step e/.

12. An L-homoraphanusamic acid prepared according to the process described in claims 1 to 11.
